(19) Europäisches Patentamt
European Patent Office
Office européen des brevets

(11) EP 0 783 530 B1

(12) **FASCICULE DE BREVET EUROPEEN**

(45) Date de publication et mention de la délivrance du brevet:
**12.08.1998 Bulletin 1998/33**

(21) Numéro de dépôt: **95932804.8**

(22) Date de dépôt: **28.09.1995**

(51) Int Cl.[6]: **C08F 2/38**, B01F 17/00, A61K 7/48, A61K 7/043

(86) Numéro de dépôt international:
**PCT/FR95/01252**

(87) Numéro de publication internationale:
**WO 96/10044 (04.04.1996 Gazette 1996/15)**

(54) **VERNIS A ONGLES CONTENANT DES MICROGELS**
NAGELLACK MIT MIKROGELPARTIKELN
NAIL VARNISH CONTAINING MICROGELS

(84) Etats contractants désignés:
**BE CH DE ES FR GB IT LI**

(30) Priorité: **28.09.1994 FR 9411575**
**28.09.1994 FR 9411576**
**06.04.1995 FR 9504120**

(43) Date de publication de la demande:
**16.07.1997 Bulletin 1997/29**

(73) Titulaire: **LVMH RECHERCHE**
**92752 Nanterre (FR)**

(72) Inventeurs:
- **TRANCHANT, Jean-François**
  **F-45760 Boigny-sur-Bionne (FR)**
- **RIESS, Henri-Gérard**
  **F-68200 Mulhouse (FR)**
- **MEYBECK, Alain**
  **20 ter, rue de Bezons F-92400 Courbevoie (FR)**

(74) Mandataire: **Giraud, Françoise et al**
**Cabinet Beau de Loménie**
**158, rue de l'Université**
**75340 Paris Cedex 07 (FR)**

(56) Documents cités:
**EP-A- 0 207 026**
**EP-A- 0 408 189**
**US-A- 5 298 585**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 783 530 B1

Printed by Jouve, 75001 PARIS (FR)

## Description

L'invention concerne de nouveaux vernis à ongles contenant des microgels ainsi que l'utilisation de ces microgels pour modifier les propriétés physiques desdits vernis et/ou du film obtenu.

La présente invention se situe tout particulièrement dans le domaine des vernis à ongles comprenant un milieu solvant organique essentiellement non aqueux. Dans de tels vernis à ongles, le solvant ou le mélange de solvants représente classiquement entre 50 et 80 % en poids par rapport au poids total du vernis à ongles. Les solvants organiques classiquement utilisés sont des esters légers, en particulier l'acétate d'éthyle, de propyle ou de butyle. Le milieu solvant organique peut également contenir des alcools légers, en particulier l'éthanol, l'isopropanol ou le butanol ou des cétones, en particulier l'acétone ou la méthyléthylcétone.

On désignera au sens de l'invention par vernis à ongles comprenant un milieu solvant organique essentiellement non aqueux ou plus simplement par vernis à ongles à solvants organiques, des vernis à ongles dans lesquels le solvant comprend au plus 1 % d'eau et est composé d'au moins l'un des solvants définis ci-dessus.

D'une façon générale, on utilise dans les vernis à solvants organiques des argiles organophiles pour empêcher la sédimentation des pigments. A titre d'exemple d'argiles organophiles utilisées couramment à cet effet, on citera les smectites, hectorites, bentonites et montmorillonites quaternisées. L'utilisation de ces produits présente des inconvénients bien connus des formulateurs de vernis à ongles : en effet, ils diminuent fortement l'adhésion du vernis, par ailleurs ils diminuent également sa brillance et, autre inconvénient, ils nécessitent généralement la présence de toluène dans la formulation.

D'une façon générale, dans les vernis à ongles comprenant un solvant organique, l'essentiel de l'extrait sec est dû à la présence de nitrocellulose.

Or, la demanderesse a maintenant découvert qu'une partie ou même la totalité de la nitrocellulose contenue dans les vernis à ongles à solvants organiques pouvait être remplacée par des microgels et que, par ailleurs, de façon tout à fait surprenante, la présence de microgels dans les compositions de vernis à ongles permettait une amélioration des propriétés rhéologiques du vernis et/ou du film, permettant en particulier d'éviter la précipitation des pigments. Cette remarquable propriété permet de diminuer sensiblement, voire même de supprimer la quantité d'argiles organophiles généralement utilisées à cet effet dans les vernis à ongles, mais dont les inconvénients sont bien connus, comme on l'a exposé ci-dessus.

Par ailleurs, les spécialistes de la formulation des vernis à ongles savent que, parmi les autres problèmes à résoudre, il est notamment essentiel de trouver un bon compromis permettant d'avoir un extrait sec élevé sans toutefois augmenter de façon trop importante la viscosité du produit. Ceci est particulièrement aigu lorsque l'on recherche à réaliser un vernis permettant d'obtenir un bel aspect de surface de l'ongle avec une seule couche de vernis. Un tel vernis est désigné couramment par l'homme du métier en utilisant le vocable anglais "one coat".

En effet, pour qu'un vernis de ce type puisse, en une seule couche, former un film de couvrance suffisante, il est généralement nécessaire d'augmenter la quantité de pigments. Toutefois, il faut veiller à ne pas dépasser une certaine concentration de pigments, car cela pourrait être préjudiciable à la brillance du film obtenu. On pourrait alors songer à augmenter l'épaisseur du film en augmentant la quantité de nitrocellulose et/ou de résine, mais dans ce cas, on se heurte à un problème d'étalement du vernis dû à une viscosité trop élevée. Pour tenter de régler ce problème, on a proposé de diminuer le grade de la nitrocellulose utilisée, mais alors la qualité du film diminue, il devient plus cassant et s'écaille.

Or, la demanderesse a mis en évidence que, de façon surprenante, l'incorporation d'un microgel dans une formule de vernis à ongles permettait d'augmenter l'extrait sec du vernis sans pour autant augmenter sensiblement sa viscosité.

Ainsi, la présente invention permet en particulier de fournir une solution au problème de la formulation des vernis à ongles de type "one coat" tel que défini ci-dessus.

Selon l'une de ses caractéristiques essentielles, l'invention concerne des vernis à ongles comprenant un milieu solvant organique essentiellement non aqueux contenant, outre les constituants usuels des vernis à ongles, au moins un microgel, ledit microgel n'étant pas obtenu à partir d'une microdispersion de polymère acrylique préparée par polymérisation radicalaire d'au moins un monomère acrylique en présence d'un copolymère-bloc à base de polyméthacrylate de méthyle (PMMA) et de polyacrylate de tertiobutyle (PAtBu).

Les microgels utilisables pour préparer les vernis à ongles selon l'invention pourront être tous les microgels, en particulicr les microgels tels qu'ils ont été définis par W. FUNKE et al., "Intramolecularly Crosslinked Macromolecules. Formation and Structure. Characterization and Particules Properties", Polym. Internat. 30 519 (1993). Dans cette publication, FUNKE et al. donne une définition complète des microgels en insistant sur les points suivants : la solubilité étant une caractéristique commune aux microgels et aux polymères linéaires, les microgels se distinguent des polymères linéaires ou branchés par un réseau de réticulation tridimensionnelle. Le diamiètre de leurs particules est inférieur à 100 nm et leur masse moléculaire est en général supérieure à $10^6$ g/mol. De ce fait, les microgels peuvent être dispersés pour former des solutions colloïdales transparentes ou légèrement opalescentes.

Les microgels obtenus à partir d'une microdispersion de polymère acrylique préparé par polymérisation radicalaire

d'au moins un monomère acrylique en présence d'un copolymère-bloc à base de polyméthacrylate de méthyle (PMMA) et de polyacrylate de tertiobutyl (PAtBu), ont été volontairement exclus de la liste des microgels utilisables selon l'invention pour tenir compte de la demande française N°93.11705 (FR-A-2 710 646) déposée par la demanderesse le 1er octobre 1993, portant sur des microdispersions stables et des microgels à base de polymères acryliques, ainsi que sur leur procédé d'obtention et des compositions notamment cosmétiques les composant.

Selon une variante particulièrement avantageuse de l'invention, le microgel comprend un polymère réticulé à base d'un monomère monofonctionnel dit monomère principal et d'un monomère difonctionnel agissant comme agent réticulant.

Le monomère principal sera avantageusement choisi parmi les monomères acryliques, méthacryliques, styréniques ou les esters vinyliques.

Plus précisément, ces monomères seront choisis dans la famille comprenant :

- les alkylacrylates et alkylméthacrylates présentant des groupements alkyl en C1 à C6, linéaires ou ramifiés, ou leur mélange,
- des monomères styréniques,
- des esters vinyliques présentant des groupements alkyles en C1 à C18, par exemple l'acétate de vinyle.

Les monomères difonctionnels faisant fonction d'agent réticulant seront choisis avantageusement parmi les monomères suivants :

- les monomères diacryliques ou diméthacryliques, par exemple le diméthacrylate de butanediol, le diméthacrylate d'éthylèneglycol, le diméthacrylate de diéthylèneglycol, le diméthacrylate de tétraéthylèneglycol,
- les monomères aromatiques difonctionnels, par exemple le divinylbenzène.

On choisira préférentiellement les différents monomères dans la famille des monomères de type acrylique ou méthacrylique. Le taux de réticulation des microgels représenté par la proportion de monomère difonctionnel par rapport au monofonctionnel est avantageusement compris entre 0,5 et 40 % en poids, de préférence entre 0,5 et 15 %.

De tels microgels existent, pour certains, dans le commerce. On citera en particulier les microgels commercialisés par la société NIPPON PAINT.

Ces microgels pourront également être obtenus par gonflement dans un solvant du polymère d'un microlatex préparé par polymérisation radicalaire d'un monomère monofonctionnel tel que défini précédemment, en présence d'un monomère réticulant et d'un agent tensioactif, de préférence un agent tensioactif anionique.

Pour préparer ces microgels, on préparera en particulier des solutions micellaires de tensioactifs en présence desquels les microlatex seront préparés par tout procédé de polymérisation en présence d'un amorceur organosoluble ou hydrosoluble comme décrit par W. FUNKE avec des polyesters saturés ou insaturés en particulier dans :

- "Emulsifying properties of saturated polyesters"
  H. BAUMANN, B. JOOS, W. FUNKE, Makromol. Chem., 187, 2933 (1986),

- "Saturated polyesters as emulsifiers for emulsion copolymerization of unsaturated polyester resins with styrene"
  H. BAUMANN, B. JOOS, W. FUNKE
  Makromol. Chem., 190, 83-92 (1989),

- "Reactor Microgels by Self-emulsifying Copolymerization of Unsaturated Polyester Resins with Acrylic and Methacrylic Esters"
  Makromol. Chem., 184, 755-762 (1983)
  M. MIYATA, W. FUNKE

- "Reactive Microgels by Emulsion Polymerization of Unsaturated Polyester Resins"
  Y.-Ch. YU, W. FUNKE
  Die Angewandte Makromol. Chem., 103, 187-202 (1982),

- "Surfactant Properties of Unsaturated Polyesters"
  Y.-Ch. Yu, W. FUNKE
  Die Angewandte Makromol. Chem., 103, 203-215 (1982).

Pour préparer les microlatex que l'on transforme ensuite en microgels avant leur incorporation dans les vernis selon l'invention, on peut utiliser différents tensioactifs cités dans la littérature en particulier :

- des tensioactifs classiques anioniques, en particulier du dodécylsulfate de sodium, dont les références bibliographiques sont les suivantes :

  D. KÜHNLE et W. FUNKE, "Uber die Reaktionsfähigkeit der Vinylgruppen in vernetzten Divinylbenzol-Polymeren und Styrol-Divinylbenzol-Copolymeren", Makromol. Chem., 139, 255, (1971)

  W. BEER, D. KÜHNLE et W. FUNKE, "Darstellung raümlich vernetzten Polymere aus difunktionellen Monomeren und reaktiven polyfunktionellen Microgel - und Gelpartikeln", Angew. Makromol. Chem., 23, 205 (1972)

  W. OBRECHT, U. SEITZ et W. FUNKE, "Zur Herstellung von reaktiven Microgelen durch Emulsion Polymerisation von reinen mehrfunktionellen Vinylmonomeren", Makromol. Chem., 175, 3587 (1974);

- des tensioactifs cationiques classiques comme décrit dans S. ISHIKURA, K. ISHII et R. MIZUGUSHI, "Flow and film properties of coatings containing microgels", Prog. in Org. Coat., 15, 373 (1988),

- des tensioactifs non ioniques, en particulier du polyoxyéthylène-polyoxypropylène (POE-POP), comme décrit dans EP 0 267 726,

- des mélanges de tensioactifs classiques et de tensioactifs non ioniques comme décrit dans le brevet allemand DE-3 723 274,

- des résines époxy modifiées par des zwittérions, comme décrit dans H. MURAMOTO, K. ISHII, T. MUYAZONO, S. ISHIKURA et R. MIDZUGUSHI, "Design of microgel-containing Coatings", Proceedings 13th Int. Conf. Org. Coat. Sci. Techn., 237 (1987) et dans S. ISHIKURA, "Zwitter ion Modified Epoxy Resins that serve as Emulsifier in the Microgel Forming Emulsion Polymerization Reaction", ACS Org. Coatings and Polym. Sci Proc., 48, 989 (1983),

- des polyesters saturés et insaturés comme décrit dans W. FUNKE, R. KOLITZ et W. SRAEHLE, "Emulsion Polymerization of unsaturated polyester resins", Makromol, Chem., 180, 2797 (1979), dans Y. Ch. YU et W. FUNKE, "Reactive microgels by emulsion polymerisation of unsaturated polyester resins", Angew. Makromol. Chem., 103, 187 (1982) et dans H. BAUMANN, B. JOOS et W. FUNKE, "Emulsifying properties of saturated polyesters", Makromol. Chem., 187, 2933 (1986),

- des sulfosuccinates comme décrit dans le brevet US 4 414 357.

On choisira avantageusement comme tensioactifs des tensioactifs macromoléculaires, en particulier des polyesters fonctionnalisés par des groupements carboxyliques.

Selon une autre variante particulièrement avantageuse de l'invention, le tensioactif macromoléculaire appartiendra à une première famille (I) constituée :

- des polymères fonctionnalisés répondant à la formule :

$$(P)\text{-}S\text{-}X\text{-}F \qquad (1)$$

dans laquelle :

. (P) est une chaîne polymérique à caractère hydrophobe de masse molaire moyenne en nombre comprise entre 500 et 250 000,
. S représente le soufre,
. X représente :

  - une chaîne hydrocarbonée saturée ou insaturée, linéaire ou ramifiée, comprenant de 1 à 6 atomes de carbone et substituée par au moins un groupe COOH ou $NH_2$, sous forme libre ou salifiée,

  - une chaîne peptidique constituée de 2 à 4 acides aminés, en particulier naturels,

. F représente un groupe COOH ou $NH_2$, sous forme libre ou salifiée

- et des polymères résultant de la polymérisation radicalaire d'au moins un monomère en présence d'un peptide porteur d'au moins un groupement disulfure et/ou d'au moins une fonction thiol, tel qu'un hydrolysat de kératine.

La chaîne polymérique (P) est avantageusement obtenue par polymérisation radicalaire d'un monomère acrylique ou vinylique.

Parmi les monomères acryliques, on citera tout particulièrement les acrylates, méthacrylates, éthylacrylates d'un groupe hydrocarboné en C1 à C18, saturé ou insaturé, en particulier allylique, linéaire, ramifié ou comprenant un cycle.

Un monomère préféré selon l'invention pour préparer les chaînes polymériques (P) ci-dessus est le méthacrylate de méthyle.

Parmi les monomères vinyliques, on citera notamment le styrène, l'α-méthylstyrène, les styrènes substitués, l'acrylonitrile, les esters vinyliques tels que l'acétate de vinyle.

Parmi les mélanges de monomères, on citera en particulier les mélanges d'acrylate ou de méthacrylate d'alkyle et d'acrylate ou de méthacrylate d'allyle, plus particulièrement les mélanges de méthacrylate de méthyle et de méthacrylate d'allyle. L'avantage de tels mélanges de monomères est qu'ils conduisent à des chaînes polymériques partiellement insaturées permettant d'obtenir des propriétés spécifiques des polymères liées à la présence de ces insaturations dans la chaîne polymérique.

Des produits particuliers de la famille de polymères (I) ci-dessus ont été décrits par :

. Y. YAMASHITA, Y. CHUJO, H. KOBAYASHI et KAWAKAMI dans Polym. Bull., 5, 361-366 (1981) ; cette publication décrit des macromonomères de formule générale (PMMA)SCH(COOH)$CH_2$COOH ainsi que d'autres macromonomères acryliques présentant la même fonction terminale. Tous ces macromonomères sont destinés à être utilisés dans des opérations de polycondensation ;

. Y. CHUJO, H. KOBAYASHI et Y. YAMASHITA dans J. of Polym. Sci., Part A : Polym. Chem., 27, 2007-2014 (1989); cette publication décrit des macromonomères destinés également à des opérations de polycondensation, ces macromonomères étant constitués d'une chaîne polymérique PMMA terminée par un groupement fonctionnel aromatique dicarboxylique.

Les différents travaux de YAMASHITA, CHUJO et coll.. ont porté sur certains polymères de cette famille obtenus par polymérisation radicalaire d'un monomère en présence d'acide thiomalique. Les produits utilisables selon l'invention comme agent tensioactif macromoléculaire peuvent être obtenus dans un procédé analogue à celui décrit dans les travaux de YAMASHITA, CHUJO et coll. cités précédemment, mais en utilisant différents autres agents de transfert de chaîne lors de la polymérisation radicalaire. Ces agents de transfert de chaîne étant constitués par des thiols ou des disulfures susceptibles de se décomposer pour générer des groupements thiol agissant à leur tour comme agent de transfert de chaîne.

Ainsi, selon une variante, le polymère fonctionnalisé servant de tensioactif macromoléculaire pourra résulter de la polymérisation radicalaire d'un monomère en présence d'un thiol répondant à la formule H-S-X-F ou d'un disulfure répondant à la formule F-X-S-S-X-F dans lesquelles :

- X représente:
  - une chaîne hydrocarbonée saturée ou insaturée, linéaire ou ramifiée, comprenant 1 à 6 atomes de carbone et substituée par au moins un groupe COOH ou $NH_2$, sous forme libre ou salifée
  - une chaîne peptidique constituée de 2 à 4 acides aminés, en particulier, naturels,
- F représente un groupe COOH ou $NH_2$, sous forme libre ou salifiée, ledit thiol ou disulfure agissant comme agent de transfert de chaîne lors de ladite polymérisation radicalaire, ledit (ou lesdits) monomère(s) conduisant à la formation de la chaîne polymérique (P) telle que précédemment définie.

Le principe du procédé de synthèse ci-dessus est, comme on l'a vu précédemment, directement inspiré des travaux de YAMASHITA, CHUJO et coll, qui. ont développé ce type de macromonomères dans le but de les copolymériser par polycondensation et ainsi former des copolymères greffés (voir en particulier, E.J. GOETHALS, "Telechelic Polymers Synthesis and Applications" CRC Press, Inc, 169-179 (1989)).

Le principe de cette synthèse consiste à polymériser du méthacrylate de méthyle par voie radicalaire en présence d'un agent de transfert portant les fonctions acides, en l'occurrence l'acide thiomalique, dans les conditions indiquées dans le schéma réactionnel ci-dessous:

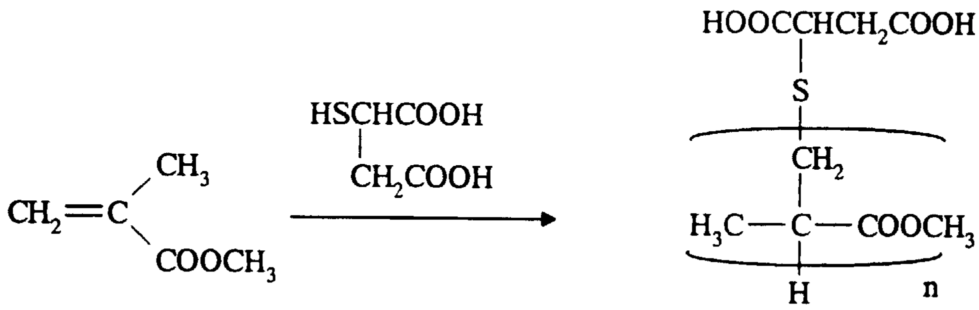


Cette réaction s'effectue en milieu solvant, par exemple THF, en présence d'un amorceur de polymérisation radicalaire, par exemple l'azobisisobutyronitrile (AIBN) à une température de l'ordre de 60°C.

Les polymères fonctionnalisés pourront avantageusement être préparés dans un procédé par analogie inspiré du schéma réactionnel ci dessus, en choisissant le(s) monomère(s) et le thiol en fonction du produit final visé.

La réaction de polymérisation radicalaire du (ou des) monomère(s) sera réalisée en milieu solvant, en présence d'un agent de polymérisation radicalaire constitué d'un amorceur organo-soluble, de préférence choisi dans la famille des amorceurs azoïques.

A titre d'exemple d'amorceur préféré, on citera l'azobisisobutyronitrile (AIBN).

La réaction a lieu en milieu solvant.

Le solvant ou mélange de solvants sera choisi en fonction de la nature du (ou des) monomère(s) à polymériser et du thiol.

Le solvant ou mélange de solvants sera choisi en fonction de la nature des réactifs. De préférence il s'agira d'un solvant ou d'un mélange de solvants capable de dissoudre l'ensemble des réactifs en présence, à savoir les monomères, le polymère formé, l'amorceur et l'agent de transfert.

Le solvant peut avoir un caractère acide, on utilisera, par exemple, l'acide acétique; il peut également avoir un caractère basique, par exemple, la diméthyléthanolamine.

La température de réaction sera avantageusement comprise entre 30°C et 120°C, mais est à ajuster en fonction des réactifs en présence. On comprend aisément qu'elle dépend de la nature de l'amorceur et de la nature du solvant.

La masse moléculaire du polymère fonctionnalisé résultant du procédé décrit ci-dessus sera contrôlée en jouant sur la quantité d'agent transféreur de chaîne introduite.

Les proportions d'amorceur, transféreur et monomère(s) peuvent être calculées d'après la relation classique connue pour le transfert de chaîne :

$$\frac{1}{DPn} = \frac{1}{DPn_0} + Cs\left(\frac{S}{M}\right)$$

où

- S/M est le rapport molaire thiol/monomère à engager
- Cs est la constante de transfert dépendant de la nature du (des) monomère(s), de l'agent de transfert, de la température et du solvant
- DPn est le degré de polymérisation du polymère que l'on veut synthétiser
- $DPn_0$ est le degré de polymérisation du polymère que l'on aurait obtenu en absence d'agent de transfert.

D'une manière générale, pour préparer les polymères fonctionnalisés répondant à la formule (1) décrite précédemment, on utilisera comme agent de transfert de chaîne un thiol répondant à la formule H-S-X-F où X et F ont les significations données précédemment.

Selon une variante de procédé, on pourra utiliser comme agent de transfert de chaîne un disulfure de formule F-X-S-S-X-F, dans la mesure où ce disulfure est susceptible de se scinder en deux radicaux F-X-S dans les conditions de la polymérisation radicalaire, ces deux radicaux agissant de façon analogue à ce qui se passe en présence du thiol correspondant H-S-X-F.

Les thiols utiles pour la préparation des composés de la famile (I) sont tous les composés répondant à la formule H-S-X-F dans laquelle :

X représente :

- une chaîne hydrocarbonée saturée ou insaturée, linéaire ou ramifiée, comprenant 1 à 6 atomes de carbones et substituée par au moins un groupe COOH ou $NH_2$, libre ou salifié
- une chaîne peptidique constituée de 2 à 4 acides aminés, en particulier, naturels,

F représente un groupe COOH ou $NH_2$, sous forme libre ou salifiée.

Les disulfures utiles pour préparer les polymères fonctionnalisés de la famille (I) sont les disulfures répondant à la formule F-X-S-S-X-F où X et F ont les significations données ci-dessus.

Des polymères fonctionnalisés particulièrement préférés pour préparer les produits de la famille (I) sont ceux dans lesquels la partie F-X comprend au moins une fonction carboxylique et au moins une fonction amine sous forme libre ou salifiée.

A titre d'exemple de tels polymères, on citera ceux dans lesquels l'agent transféreur de chaîne est la cystéine ou l'homocystéine.

On citera tout particulièrement les polymères fonctionnalisés résultant de la polymérisation radicalaire d'au moins un monomère en présence de cystéine agissant comme agent de transfert de chaîne.

Un tel polymère fonctionnalisé suivant qu'il est sous forme libre ou salifiée, répond à l'une des formules :

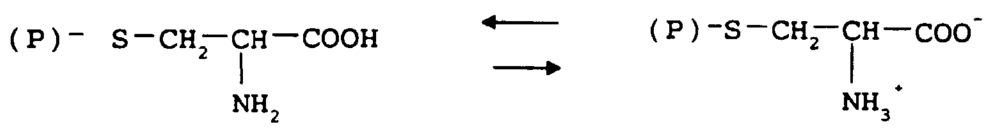

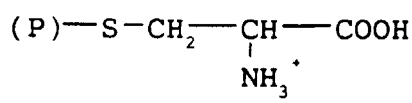

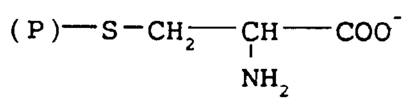

où (P) est une chaîne polymérique résultant de la polymérisation radicalaire d'au moins un monomère.

Les polymères fonctionnalisés de la famille (I) pourront également résulter de la polymérisation radicalaire d'un monomère en présence d'un thiol répondant à la formule H-S-X-F ou d'un disulfure répondant à la formule F-X-S-S-X-F dans lesquelles :

- X représente :
  - une chaîne hydrocarbonée saturée ou insaturée, linéaire ou ramifiée, comprenant 1 à 6 atomes de carbone et substituée par au moins un groupe COOH ou $NH_2$, sous forme libre ou salifée
  - une chaîne peptidique constituée de 2 à 4 acides aminés, en particulier, naturels,
- F représente un groupe COOH ou $NH_2$, sous forme libre ou salifiée, ledit thiol ou disulfure agissant comme agent de transfert de chaîne lors de ladite polymérisation radicalaire, ledit (ou lesdits) monomère(s) conduisant à la formation de la chaîne polymérique (P) telle que précédemment définie.

Les chaînes polymériques (P) définies précédemment ont de préférence une masse moyenne en nombre inférieure à 20 000, de préférence encore comprise entre 500 et 10 000.

On choisira de préférence parmi les produits décrits ci-dessus ceux dans lesquels la partie F-X comprend au moins une fonction carboxylique et au moins une fonction amine sous forme libre ou salifiée.

A titre d'exemples de polymères fonctionnalisés de la famille (I), on citera le produit de formule :

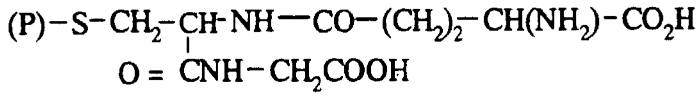

et plus particulièrement le polymère fonctionnalisé obtenu par polymérisation radicalaire d'un monomère conduisant à la chaîne polymérisée telle que définie précédemment en présence d'un agent de transfert de chaîne constitué par du gluthation.

Selon d'autres variantes avantageuses de l'invention, on réalisera la polymérisation radicalaire du monomère conduisant à la formation de la chaîne polymérique P précédemment définie en présence de cystéine ou d'homocystéine, qui agira comme agent de transfert de chaîne. On pourra également utiliser comme agent de transfert de chaîne un peptide porteur d'au moins un groupement disufure et/ou d'au moins une fonction thiol, tel qu'un hydrolysat de kératine.

Selon une autre variante de l'invention, l'agent tensioactif macromoléculaire pourra être un polymère fonctionnalisé en bout de chaîne répondant à la formule

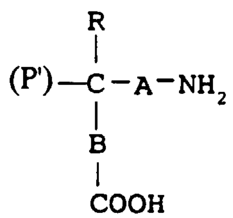

(II)

dans laquelle :

- la chaîne polymérique (P') est une chaîne avantageusement hydrophobe obtenue par polymérisation radicalaire d'au moins un monomère, et dont la masse molaire moyenne en nombre est comprise entre 500 et 250 000, de préférence inférieure à 20 000, de préférence comprise entre 500 et 10 000,
- R représente un atome d'hydrogène ou une chaîne hydrocarbonée comprenant 1 à 8 atomes de carbone, linéaire ou ramifiée, éventuellement substituée par au moins un groupe choisi parmi $CO_2H$, $NH_2$, OH ou un groupe phényle, lui-même éventuellement substitué,
- A et B, identiques ou différents, représentent chacun une liaison simple, une chaîne hydrocarbonée saturée ou insaturée, linéaire ou ramifiée comprenant de 1 à 16 atomes de carbone, pouvant contenir une fonction amide, une chaîne peptidique, comprenant 2 à 4 acides aminés, en particulier, naturels,
- les groupements COOH et/ou $NH_2$ étant libres ou salifiés,

les monomères ou mélanges de monomères constituant la chaîne (P') sont les mêmes que ceux constituant la chaîne (P) des produits de la formule (I) précédemment définie.

Ce polymère de formule (II) sera avantageusement choisi parmi les produits suivants :

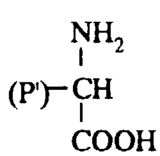

(3)

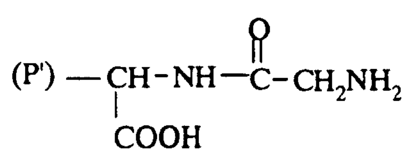

(4)

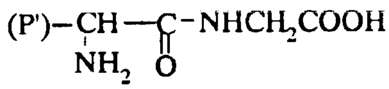 (5)

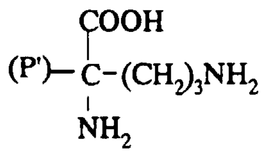 (6)

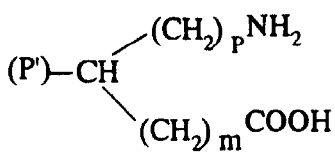 (7)

dans lesquelles :

(P') est une chaîne polymérique telle que précédemment définie,
m et p sont des entiers compris entre 0 et 11 et dont la somme est comprise entre 2 et 11,
ou d'un polymère fonctionnalisé obtenu par polymérisation radicalaire d'au moins un monomère en présence d'un acide aminé ou d'un dérivé d'acide aminé répondant à la formule :

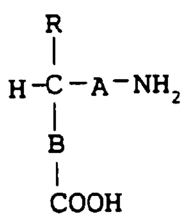 ( 2 )

dans laquelle R, A et B ont les significations données précédemment.

Les produits de la formule (II) peuvent être obtenus par polymérisation radicalaire d'au moins un monomère conduisant à la formation de la chaîne polymérique hydrophobe (P') en présence d'un acide aminé ou d'un dérivé d'acide aminé répondant à la formule (2) ci-dessous :

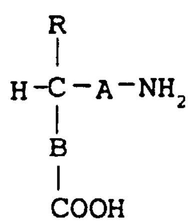 ( 2 )

agissant comme agent de transfert de chaîne lors de ladite polymérisation radicalaire.

Dans la formule (2) ci-dessus, les groupements R, A et B ont les significations données pour les mêmes groupements dans la formule (II).

Le rôle d'agent de transfert de chaîne du produit de formule (2) dans la polymérisation radicalaire est rendu possible du fait du caractère labile de l'hydrogène porté par le carbone dans la formule (2) ci-dessus.

Ce procédé qui met en oeuvre un acide aminé ou un dérivé d'acide aminé comme agent de transfert de chaîne lors de la polymérisation radicalaire d'au moins un monomère conduisant à la formation d'une chaîne polymérique (P')

est inspiré par analogie du procédé décrit dans les travaux de YAMASHITA, CHUJO et coll., cités précédemment, en remplaçant le thiol par un acide aminé ou un dérivé d'acide aminé présentant un atome d'hydrogène labile.

Plus précisément, comme cela a été signalé précédemment, la synthèse de PMMA fonctionnalisés par des groupements acides carboxyliques est déjà connue. YAMASHITA, CHUJO et coll. ont développé ce type de macromonomères dans le but de les copolymériser par polycondensation et ainsi former des copolymères greffés (voir en particulier, E.J. GOETHALS, "Telechelic Polymers Synthesis and Applications" CRC Press, Inc, 169-179 (1989)).

Le principe de cette synthèse consiste à polymériser du méthacrylate de méthyle par voie radicalaire en présence d'un agent de transfert portant les fonctions acides, en l'occurrence l'acide thiomalique, dans les conditions indiquées dans le schéma réactionnel donné précédemment.

Les microgels, en particulier ceux obtenus à partir de microlatex préparés en s'inspirant des différentes publications citées précédemment de W. FUNKE, sont d'une façon générale introduits dans une composition de vernis à ongles après avoir été préalablement gonflés dans un solvant organique, solvant du polymère consititutif dudit microlatex.

Selon une variante particulièrement avantageuse de l'invention, on utilise comme solvant organique pour gonfler le microlatex et le transformer en microgel l'un des solvants constitutifs du milieu solvant du vernis à ongles. Ce solvant sera avantageusement l'acétate d'éthyle ou l'acétate de butyle.

Les particules constitutives du microgel ont à l'état gonflé un diamètre avantageusement compris entre 10 et 300 nm, de préférence entre 20 et 150 nm, de préférence encore entre 30 et 100 nm.

Le microgel à l'état gonflé constituera avantageusement une partie au moins de la matière sèche comprise dans le vernis à ongles.

D'une façon générale, les vernis à ongles selon l'invention contiendront de 1 à 30 % en poids de microgels, de préférence de 5 à 20 %.

Les essais réalisés par la demanderesse ont montré que les microgels pouvaient avantageusement remplacer au moins partiellement la nitrocellulose présente dans les compositions de vernis à ongles, permettant ainsi d'obtenir des compositions qui, à extrait sec égal, présentent des viscosités suffisamment peu élevées pour permettre un étalement du vernis dans de bonnes conditions.

Par ailleurs, d'autres essais effectués par la demanderesse ont montré que les microgels permettaient également de remplacer avantageusement au moins une partie des argiles organophiles présentes dans les vernis à ongles, sans toutefois nuire à la stabilité de la dispersion des pigments au sein de la composition du vernis à ongles. Le remplacement au moins partiel des argiles organophiles permet au vernis selon l'invention de présenter une meilleure adhésion sur l'ongle.

Ainsi donc, l'invention concerne en particulier des vernis à ongles dans lesquels l'extrait sec est compris entre 20 et 50 % en poids, de préférence entre 25 et 35 % en poids.

L'invention permet d'obtenir notamment des vernis à ongles présentant une quantité élevée d'extrait sec, par exemple pour préparer des vernis à ongles de type "one coat". L'extrait sec pourra atteindre de 30 à 50 % en poids du vernis.

Par ailleurs, ces vernis à ongles contiennent de 0 à 30 % en poids de nitrocellulose, de préférence de 0 à 20 %, la teneur en nitrocellulose étant d'autant plus basse que la teneur en microgel est plus élevée.

D'autre part, les vernis à ongles de l'invention contiennent de 0 à 3 % en poids d'argile organophile, de préférence de 0 à 1,5 %, de préférence encore de 0 à 0,5 %. Là encore, on constate que la teneur en argile organophile peut être d'autant plus abaissée que la teneur en microgel est plus élevée.

Ainsi donc, l'utilisation des microgels dans les compositions des vernis à ongles de l'invention constitue un moyen particulièrement efficace pour modifier les propriétés physiques de ces compositions, ainsi que celles du film formé lors de l'étalement de la composition.

Plus précisément, l'utilisation des microgels dans les compositions de vernis à ongles permet, en jouant sur l'extrait sec et la viscosité de la composition, de régler à volonté la rhéologie de ces compositions.

Elle permet également, comme cela a été mis en évidence par des essais d'étirement du film formé après étalement du vernis à ongles, de modifier la plasticité de ce film en jouant sur la teneur en microgel dans le film.

Par ailleurs, des essais également réalisés par la demanderesse ont montré qu'en jouant sur la taille et la nature du microgel, ainsi que sur le degré de réticulation, on pouvait modifier la brillance du vernis obtenu.

Les exemples, qui suivent, sont donnés à titre purement illustratif de l'invention.

## EXEMPLES

Sauf indications contraires, les proportions indiquées dans les exemples ci-dessous sont exprimées en pourcentage en poids.

I - Exemple de composition de vernis à ongles

Dans tous les exemples ci-dessous de compositions complètes de vernis à ongles incorporant des microgels, on utilise une méthode de l'art antérieur, bien connue de l'homme de l'art, on prépare les vernis à ongles à partir de "solutions colorantes" de différentes teintes, que l'on mélange avec une base pour vernis à ongles.

Ces "solutions colorantes" sont en fait des dispersions de pigments dans une base contenant de la nitrocellulose, cette base pouvant être la même que celle utilisée pour la formulation finale du vernis. De préférence, les pigments sont préalablement broyés dans un solvant, tel que l'acétate de butyle, au moyen d'un broyeur approprié tel que, par exemple, un broyeur à billes de type Dyno-mill.

Les broyés sont incorporée à une base nitrocellulosique "diluante" pour préparer différentes solutions colorantes, chacune ayant sa propre teinte selon la nature et la concentration du pigment qu'elle contient.

Par exemple, la composition de la base "diluante" est la suivante :

| | | |
|---|---|---|
| - nitrocellulose | 10 à 30 %, | par exemple 15 % |
| - Lustralite®(arylsulfonamide) | 8 à 15 %, | par exemple 10 % |
| - dibutyl-phtalate | 4 à 7 %, | par exemple 5 % |
| - Néocryl®(résine acrylique) | 0 à 5 %, | par exemple 2 % |
| - acétate de butyle | 5 à 50 %, | par exemple 18 % |
| - acétate d'éthyle | 5 à 50 %, | par exemple 17 % |
| - bentonite | 0,8 à 1,5 %, | par exemple 1 % |
| - toluène | 0 à 30 %, | par exemple 25 % |
| - isopropanol | 4 à 13 %, | par exemple 7 % |
| | 100% | 100% |

La quantité de broyé introduite dans la base diluante est telle que la concentration en pigment dans la solution colorante est généralement inférieure ou égale à 20 % environ.

Suivant la teinte souhaitée de la composition finale du vernis à ongles, on introduit dans une base, telle que la base ci-dessus, différentes solutions colorantes à différentes concentrations. La teneur en pigment du vernis final est en général de l'ordre de 2 à 4 %.

I.1. Vernis à ongles contenant un microgel acryclique commercial

On utilise pour la préparation d'un vernis à ongles un microgel commercial vendu par la société NIPPON PAINT, et préalablement gonflé dans de l'acétate de butyle. Ce microgel est constitué de particules de 53 nm de diamètre. Il est constitué à base de méthylméthacrylate de méthyle et présente un taux de réticulant de 0,25 mol/g.

On prépare deux vernis à ongles notés a et b contenant les proportions suivantes:

| | VERNIS a | VERNIS b |
|---|---|---|
| - nitrocellulose | 15 %, | 10 % |
| - Lustralite®(arylsulfonamide) | 10 %, | 10 % |
| - dibutyl-phtalate | 5 %, | 5 % |
| - Néocryl®(résine acrylique) | 2 %, | 2 % |
| - acétate de butyle | 18 %, | 18 % |
| - acétate d'éthyle | 15 %, | 15 % |
| - bentonite | 0,5 %, | 0,5 % |
| - toluène | 15 %, | 15 % |
| - isopropanol | 6,5 %, | 4,5 % |
| - microgel | 13 % | 20 % |

I.2. Vernis à ongles contenant des microgels obtenus à partir de tensioactif de la famille I

a) Microgel a

On prépare un polymère fonctionnalisé par l'acide thiomalique présentant une chaîne PMMA de masse théorique moyenne en nombre de 1 000 de la façon suivante.

Les proportions à cngager en amorceurs, transféreurs et monomère ont été calculées à partir des résultats de YAMASHITA, CHUJO et coll. d'après la formule :

$$\frac{1}{DPn} = \frac{1}{DPn_0} + Cs(\frac{S}{M})$$

Pour préparer un polymère fonctionnalisé ayant une $M_n$ théorique de 1000 : 1,58g d'AIBN, 33,47 g d'acide thiomalique et 100 g de MMA sont solubilisées dans 350 ml de THF (tétrahydrofuranne).

Cette solution est placée dans un réacteur double enveloppe équipé d'une ancre d'agitation, d'un réfrigérant et d'une circulation d'azote et est chauffée à 60°C durant 2 h 30.

Le mélange recueilli est précipité dans l'éther de pétrole 1 à 2 fois afin d'éliminer les restes de MMA, d'AIBN et de ses produits de décomposition. Séché, il est ensuite solubilisé dans l'acétone et reprécipité dans l'eau plusieurs fois (2 fois) afin de le débarrasser du thiol et du disulfure. Un dosage des fonctions acides avant et après précipitation montre l'efficacité et par conséquent la nécessité de cette dernière purification.

La masse moléculaire en nombre est de :

1030 (déterminée par osmométrie à tension de vapeur)
960 (déterminée par GPC, chromatographie par perméation de gel).

5 g du polymère ci-dessus sont ensuite solubilisés dans 250 ml de THF.

On détermine par dosage acido-basique en milieu THF le nombre de moles de fonctions -COOH à neutraliser, : ici 7,87 $10^{-3}$ mol et on ajoute un excès de base soit 8,2 $10^{-3}$mol.

- 0,73 g de DMEA (diméthyléthanolamine) sont solubilisées dans 500 g d'eau.

La solution organique de polymère est ajoutée lentement, sous agitation à la solution aqueuse d'amine.

Le mélange obtenu est distillé sous pression réduite afin d'éliminer le THF, puis filtré.

On a alors une solution micellaire présentant un effet Tyndall.

La taille des micelles mesurée en COULTER N4 (diffusion de la lumière) est d'environ 4,6 ± 1,4 nm.

Ce type de micelles peut être engagé dans la préparation de microlatex.

On donne ci-dessous un exemple dc formulation d'un tel latex :

- 150 g d'eau
- 0,9 g de tensio-actif (polymère fonctionnalisé neutralisé)
- 2,7 g de MMA
- 0,3 de BDMA (1,4-butanediol diméthacrylate)
- 0,075 g de persulfate de potassium.
- 0,046 g de $NaHCO_3$ comme tampon.

La polymérisation est effectuée dans un réacteur à double enveloppe, muni d'une ancre d'agitation, d'un réfrigérant et d'une arrivée d'azote.

La solution micellaire est introduite dans le réacteur où elle est chauffée à 65°C et désoxygénée pendant une heure par bullage d'azote. A cette solution agitée à 250tr/min., on ajoute le mélange de monomères. L'amorceur est introduit sous forme de solution aqueuse après 15 à 20 minutes d'émulsification

On laisse alors la polymérisation se dérouler sous atmosphère d'azote pendant environ 20 heures.

Dans le cas de la formulation précédente, la taille du microlatex obtenue est mesurée au COULTER N4 ; les résultats sont les suivants :

- diamètre moyen en poids : Dw = 28,6 ± 1,4 nm
- diamètre moyen en nombre : Dn = 23,7 ± 3,2 nm

Le microlatex ainsi préparé en utilisant le polymère fonctionnalisé comme agent tensio-actif présente différents avantages résumés ci-dessous :

La chaîne hydrophobe est de même nature que le coeur de la particule. Il y a donc apport de matière en plus du rôle tensio-actif.

Ce microlatex est réticulé. Il peut donc être transféré en milieu solvant pour donner un microgel.

Le microlatex cité précédemment a été séché puis redispersé dans l'acétate de butyle.

La taille du microgel obtenu est mesurée au COULTER N4 :

Dw = 36,9 ± 0,8 nm.

On remarque le gonflement dcs particules dans l'acétate de butyle par rapport à l'eau.

b) Microgel b

On prépare un poly(méthacrylate de méthyle-co-méthacrylate d'allyle) fonctionnalisé par l'acide thiomalique de la façon suivante :

0,32 g d'AIBN, 0,69 g d'acide thiomalique, 4 g de méthacrylate d'allyle et 16 g de MMA sont solubilisés dans 70 ml de THF.

Cette solution placée dans un réacteur double-enveloppe équipé d'une ancre d'agitation, d'un réfrigérant et d'une circulation d'azote, est chauffée à 60°C durant 2 h 30.

Le mélange recueilli est précipité dans l'éther de pétrole. Séché, il est ensuite solubilisé dans l'acétone et réprécipité dans l'eau (2 fois).

La masse moléculaire en nombre est de :

1420 (déterminée par GPC)

1500 (déterminée par VPO, osmométrie à tension de vapeur).

Puis, 2,5 g de ce polymère sont solubilisés dans 250 ml de THF.

D'après le dosage acido-basique en milieu THF du polymère par de la potasse, il y a 2,98 $10^{-3}$ moles de -COOH à neutraliser. On neutralise par un excès de base, ici 3,37 $10^{-3}$ moles, soit 0,3 g de DMEA.

Ces 0,3 g de DMEA sont solubilisés dans 250 ml d'eau.

La solution organique de polymère est ajoutée lentement, sous agitation à la solution aqueuse d'amine. Le mélange obtenu est distillé sous pression réduite afin d'éliminer le THF, puis filtré.

La taille des micelles mesurée au COULTER N4 est d'environ 5,5 ± 0,6 nm.

Ce type de micelles peut être engagé dans la préparation de microlatex.

On prépare comme dans l'exemple b et avec les mêmes conditions de synthèse un microlatex.

La taille du microlatex ainsi obtenu est mesurée au COULTER N4 :

$$Dw = 27,4 \pm 1,6nm$$

$$Dn = 22,5 \pm 2,5 \text{ nm.}$$

Les mêmes avantages que dans l'exemple précédent sont obtenus avec ce polymère fonctionnalisé :

- Apport de matière comme dans l'exemple précédent
- La chaîne hydrophobe du polymère comporte des groupements allyliques pendants capables de copolymériser avec le coeur de particule.

Le tensioactif est alors lié chimiquement à la particule.

Le microlatex réticulé cité précédemment a été séché puis redispersé dans l'acétate de butyle.

La taille du microgel (b) obtenu est mesurée au COULTER N4 :

$$Dw = 38,8 \pm 2,0 \text{ nm.}$$

c) Microgel c

On prépare un polymère constitué par du PPMA de Mn égale à 3380 fonctionnalisé en bout de chaîne par de la cystéine

On procède comme dans les excmples a et b ci-dessus avec les conditions opératoires suivantes :

- 40 g de MMA
- 8,76 g de cystéine
- 0,63 g d'AIBN
- 320 g d'acide acétique
- 80 g d'eau
- T = 60°C
- Durée de polymérisation : 5 h.

Puis, on solubilise les 2,5 g de polymère ci-dessus dans 250 ml de THF.

On détermine par dosage acido-basique en milieu THF le nombre de moles de fonctions -COOH à neutraliser : ici $7,4.10^{-4}$ moles et on ajoute un excès de base, soit environ $8,5.10^{-4}$ moles.

- $8,5.10^{-4}$ moles de potasse (KOH) sont diluées dans 250 ml d'eau.
- La solution organique de polymère est ajoutée lentement, sous agitation à la solution aqueuse de potasse.
- Le mélange obtenu est distillé sous pression réduite afin d'éliminer le THF, puis filtré sur Goosh de posoristé N°4.
- La solution résultante peut être engagée dans la préparation de microlatex.

On prépare un microlatex répondant à la formulation suivante :

- 150 g d'eau
- 0,35 g de tensioactif (polymère fonctionnalisé neutralisé)
- 2,7 g de MMA
- 0,3 g de BDMA
- 2 x 0,075 g d'AIBN
- 0,046 g de $NaHCO_3$
- La polymérisation est effectuée dans un réacteur à double enveloppe, muni d'une ancre d'agitation, d'un réfrigérant et d'une arrivée d'azote.
- L'émulsion constituée de l'eau, du tensioactif, des monomères et de $NaHCO_3$ est préparée sous forte agitation et désoxygénée par bullage d'azote.
- 0,075 g d'AIBN solubilisée dans 2 g d'acétone sont introduits après 15 à 20 minutes.
- Après 4 heures, 0,075 g d'AIBN dans 2 g d'acétone sont réintroduits dans le réacteur.
- On laisse alors la polymérisation se dérouler sous atmosphère d'azote durant environ 20 heures.

Dans le cas de la formulation précédente, la taille du microlatex obtenu est mesurée au COULTER N4. Les résultats sont les suivants :

- diamètre moyen en poids : Dw = 63,0 ± 1,7 nm
- diamètre moyen en nombre : Dn = 59,7 ± 2,5 nm

Le microlatex ainsi préparé en utilisant le polymère fonctionnalisé comme agent tensio-actif présente différents avantages résumés ci-dessous :

- La chaîne hydrophobe est de même nature que le coeur de la particule. Il y a donc apport de matière en plus du rôle tensioactif.
- Ce microlatex est réticulé. Il peut donc être transféré en milieu solvant pour donner un microgel.

On transfert ce microlatex dans l'acétate de butyle. On otbient le microgel (c).

d) On prépare trois vernis à onglcs en introduisant dans la base citée précédemment 5 % en poids de chacun des microgels a, b, c décrits ci-dessus.

II - Mise en évidence de l'influence des microgels sur les propriétés physiques des vernis à ongles

II.1. Influence sur la rhéologie :

On réalise à l'aide de microgcl commercial utilisé dans l'exemple I.1. les compositions représentées dans le tableau I ci-dessous avec leur seuil d'écoulement.

TABLEAU I

| Microgel | Nitrocellulose | Solvant | Seuil d'écoulement dyne/cm |
|---|---|---|---|
| 0,93 | 17,6 | 81,5 | 0,17 |
| 7,8 | 14 | 78,3 | 0,23 |
| 10,8 | 13 | 76,3 | 2,85 |
| 15,4 | 10,8 | 73,8 | 3,24 |
| 20 | 9 | 71 | 94,7 |
| 26,9 | 5,9 | 67,2 | 755 |

Il ressort que pour obtenir des résultats satisfaisants d'un point de vue comportement rhéologique, il faut incorporer des concentrations de microgel de 15 à 20 %. Le produit obtenu est visqueux et filant quand on utilise le pinceau.

Dans la mesure où il apparaît que la viscosité augmente relativement peu avec les microgels à faible concentration, l'utilisation de microgels permet d'augmenter l'extrait sec sans trop faire varier la viscosité.

Le Tabelau II illustre clairement ce phénomène.

TABLEAU II

| Composition | Viscosité en mPa.s |
|---|---|
| 20 % Nitrocellulose E35 | 600 |
| 15 % Nitrocellulose E35<br>5 % Microgel | 200 |
| 15 % Nitrocellulose E35 | 150 |

II. Influence du microgel sur la plasticité des films nitrocellulosiques.

On réalise des essais comparatifs de rupture de film après étirement.

Pour cela, on prépare trois compositions de vernis à ongles (notées a, b, c) :

- la composition a est réalisée en utilisant une base diluante telle que définie à l'exemple I, mais sans dibutylphtalate ;
- la composition b est réalisée avec la même base, mais contenant une quantité de dibutylphtalate représentant 25

% de l'extrait sec ;

- la composition c est obtenue, en remplaçant dans la composition b, le dibutylphtalate par la même quantité du microgel commercial utilisé dans l'exemple I.1.

Les essais d'étirement à la rupture du film montrent que le microgel confère au film la même plasticité que le dibutylphtalate (les valeurs d'étirement à la rupture différent de moins de 5 %).

**Revendications**

1. Vernis à ongles contenant un milieu solvant organique essentiellement non aqueux, caractérisé en ce qu'il contient, outre les constituants usuels des vernis à ongles, au moins un microgel, ledit microgel n'étant pas obtenu à partir d'une microdispersion de polymère acrylique préparée par polymérisation radicalaire d'au moins un monomère acrylique en présence d'un copolymère-bloc à base de polyméthacrylate de méthyle (PMMA) et de polyacrylate de tertiobutyle (PAtBu).

2. Vernis à ongles selon la revendication 1, caractérisé en ce que ledit microgel comprend un polymère réticulé à base d'un monomère monofonctionnel dit monomère principal et d'un monomère difonctionnel agissant comme réticulant.

3. Vernis à ongles selon la revendication 2, caractérisé en ce que ledit monomère principal est un monomère acrylique ou méthacrylique, styrénique ou un ester vinylique présentant un groupement alkyle en C1 à C18.

4. Vernis à ongles selon l'une des revendications 2 ou 3, caractérisé en ce que le monomère réticulant est un monomère diacrylique ou diméthacrylique, ou un monomère aromatique difonctionnel.

5. Vernis à ongles selon l'une des revendications 2 à 4, caractérisé en ce que ledit microgel est obtenu par gonflement dans un solvant du polymère d'un microlatex préparé par polymérisation radicalaire d'un monomère monofonctionnel en présence d'un monomère réticulant et d'un agent tensioactif, de préférence un agent tensioactif anionique.

6. Vernis ongles selon la revendication 5, caractérisé en ce que l'agent tensioactif est un agent tensioactif macromoléculaire.

7. Vernis ongles selon l'une des revendications 5 ou 6, caractérisé en ce que ledit agent tensioactif est un polyester fonctionnalisé par des groupements carboxyliques.

8. Vernis ongles selon l'une des revendications 5 ou 6, caractérisé en ce que ledit agent tensioactif est un polymère fonctionnalisé répondant à la formule :

$$(P) - S - X - F \qquad (1)$$

dans laquelle :

- (P) est une chaîne polymérique hydrophobe de masse molaire moyenne en nombre comprise entre 500 et 250 000,
  S représente le soufre,
- X représente :
  - une chaîne hydrocarbonée saturée ou insaturée, linéaire ou ramifiée, comprenant 1 à 6 atomes de carbone et substituée par au moins un groupe COOH ou $NH_2$, sous forme libre ou salifiée
  - une chaîne peptidique constituée de 2 à 4 acides aminés, en particulier, naturels,

  F représente un groupe COOH ou $NH_2$, sous forme libre ou salifiée

ou un polymère résultant de la polymérisation radicalaire d'au moins un monomère en présence d'un peptide porteur d'au moins un groupement disulfure et/ou d'au moins une fonction thiol, tel qu'un hydrolysat de kératine.

**9.** Vernis ongles selon la revendication 8, caractérisé en ce que ledit polymère fonctionnalisé résulte de la polymérisation radicalaire d'un monomère en présence d'un thiol répondant à la formule H-S-X-F ou d'un disulfure répondant à la formule F-X-S-S-X-F dans lesquelles :

- X représente :
  - une chaîne hydrocarbonée saturée ou insaturée, linéaire ou ramifiée, comprenant 1 à 6 atomes de carbone et substituée par au moins un groupe COOH ou $NH_2$, sous forme libre ou salifiée
  - une chaîne peptidique constituée de 2 à 4 acides aminés, en particulier, naturels,
- F représente un groupe COOH ou $NH_2$, sous forme libre ou salifiée, ledit thiol ou disulfure agissant comme agent de transfert de chaîne lors de ladite polymérisation radicalaire, ledit (ou lesdits) monomère(s) conduisant à la formation de la chaîne polymérique (P) telle que définie dans la revendication 8.

**10.** Vernis à ongles selon l'une des revendications 8 ou 9, caractérisé en ce que la chaîne polymérique (P) a une masse molaire moyenne en nombre de préférence inférieure à 20 000 et de préférence comprise entre 500 et 10 000.

**11.** Vernis à ongles selon l'une des revendications 8 à 10, caractérisé en ce que la partie F-X, F et X ayant les significations données dans la revendication 8, comprend au moins une fonction carboxylique et au moins une fonction amine, sous forme libre ou salifiée.

**12.** Vernis à ongles selon l'une des revendications 8 à 11, caractérisé en ce que ledit polymère fonctionnalisé répond à la formule :

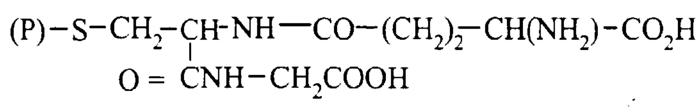

**13.** Vernis à ongles selon la revendication 12, caractérisé en ce que ledit polymère fonctionnalisé est obtenu par polymérisation radicalaire d'un monomère conduisant à la chaîne polymérisée telle que définie dans la revendication 8 en présence d'un agent de transfert de chaîne constitué par du gluthation.

**14.** Vernis à ongles selon l'une des revendication 8 à 11, caractérisé en ce que ledit polymère fonctionnalisé est obtenu par polymérisation radicalaire d'au moins un monomère en présence de cystéine ou d'homocystéine agissant comme agent de transfert de chaîne.

**15.** Vernis à ongles selon l'une des revendications 8 à 11, caractérisé en ce que ledit polymère fonctionnalisé est obtenu par polymérisation radicalaire d'au moins un monomère en présence d'un peptide porteur d'au moins un groupement disulfure et/ou d'au moins une fonction thiol, tel qu'un hydrolysat de kératine.

**16.** Vernis à ongles selon l'une des revendications 5 ou 6, caractérisé en ce que ledit agent tensioactif est un polymère fonctionnalisé en bout de chaîne répondant à la formule :

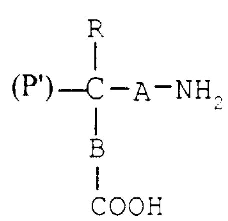

(II)

dans laquelle :

- la chaîne polymérique (P') est une chaîne hydrophobe obtenue par polymérisation radicalaire d'au moins un monomère, et dont la masse molaire moyenne en nombre est comprise entre 500 et 250 000, de préférence inférieure à 20 000, de préférence comprise entre 500 et 10 000,
- R représente un atome d'hydrogène ou une chaîne hydrocarbonée comprenant 1 à 8 atomes de carbone, linéaire ou ramifiée, éventuellement substituée par au moins un groupe choisi parmi $CO_2H$, $NH_2$, OH ou un groupe phényle, lui-même éventuellement substitué,
- A et B, identiques ou différents, représentent chacun une liaison simple, une chaîne hydrocarbonée saturée ou insaturée, linéaire ou ramifiée comprenant de 1 à 16 atomes de carbone, pouvant contenir une fonction amide, une chaîne peptidique, comprenant 2 à 4 acides aminés, en particulier, naturels,
- les groupements COOH et/ou $NH_2$ étant libres ou salifiés.

**17.** Vernis à ongles selon la revendication 16, caractérisé en ce que ledit polymère fonctionnalisé répond à l'une des formules suivantes :

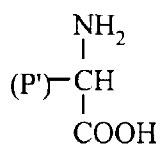

(3)

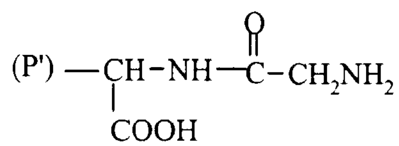

(4)

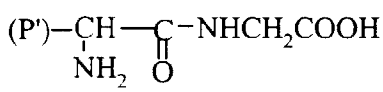

(5)

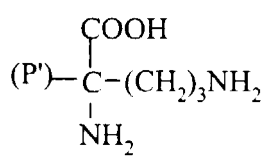

(6)

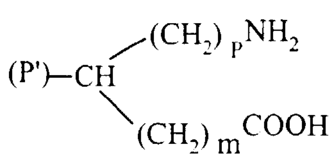

(7)

dans lesquelles :

(P') est une chaîne polymérique telle que définie à la revendication 17,
m et p sont des entiers compris entre 0 et 11 et dont la somme est comprise entre 2 et 11,

ou est un polymère fonctionnalisé obtenu par polymérisation radicalaire d'au moins un monomère en présence d'un acide aminé ou d'un dérivé d'acide aminé répondant à la formule :

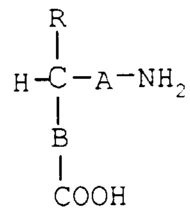

(2)

dans laquelle R, A et B ont les significations données dans la revendication 17.

**18.** Vernis à ongles selon l'une des revendications 8 à 17, caractérisé en ce que ladite chaîne polymérique résulte de la polymérisation radicalaire d'au moins un monomère acrylique ou vinylique.

**19.** Vernis à ongles selon l'une des revendications 1 à 18, caractérisé en ce que ledit microgel est constitué de microparticules qui à l'état gonflé ont un diamètre compris entre 10 et 300 nm, de préférence entre 20 et 150 nm, de préférence encore entre 30 et 100 nm.

**20.** Vernis à ongles selon l'une des revendications 1 à 19, caractérisé en ce qu'il contient de 1 à 30 % en poids de microgel, de préférence de 5 à 20 %.

**21.** Vernis à ongles selon la revendication 20, caractérisé en ce que l'extrait sec représente de 20 à 50 % en poids.

**22.** Vernis à ongles selon l'une des revendications 1 à 21, caractérisé en ce qu'il contient de 0 à 30 % en poids de nitrocellulose, de préférence de 0 à 20 %.

**23.** Vernis à ongles selon l'une des revendication 1 à 22, caractérisé en ce qu'il contient de 0 à 3 % en poids d'argile organophile, de préférence de 0 à 1,5 %, de préférence encore de 0 à 0,5 %.

**24.** Utilisation d'un microgel tel que défini dans l'une des revendications précédentes comme agent destiné à modifier les propriétés physiques d'une composition de vernis à ongles à solvants essentiellement organiques et/ou les propriétés du film obtenu lors de l'étalement de ladite composition.

**25.** Utilisation selon la revendication 24, caractérisée en ce que ledit agent est destiné à améliorer la plasticité dudit film.

**26.** Utilisation selon la revendication 24, caractérisée en ce que ledit agent est destiné à régler la rhéologie de ladite composition.

**Patentansprüche**

**1.** Nagellack, welcher ein im wesentlichen nicht-wässeriges, organisches Lösungsmittelmedium enthält, dadurch gekennzeichnet, daß er, außer den üblichen Bestandteilen von Nagellacken, zumindest ein Mikrogel enthält, wobei das Mikrogel nicht aus einer Mikrodispersion von Acryl-Polymer erhalten wird, das durch Radikalpolymerisation zumindest eines Acryl-Monomers in Anwesenheit eines Blockcopolymers auf der Basis von Polymethylmethacrylat (PMMA) und Poly-tert.butylacrylat (PtBuA) hergestellt wird.

**2.** Nagellack nach Anspruch 1, dadurch gekennzeichnet, daß das Mikrogel ein vernetztes Polymer auf der Basis eines monofunktionellen Monomers, das als Hauptmonomer bezeichnet wird, und eines difunktionellen Monomers, das als Vernetzungsmittel wirkt, umfaßt.

**3.** Nagellack nach Anspruch 2, dadurch gekennzeichnet, daß das Hauptmonomer ein Acryl- oder Methacryl-Monomer, ein Styrol-Monomer oder ein Vinylester mit einer $C_1$-$C_{18}$-Alkyl-Gruppe ist.

**4.** Nagellack nach einem der Ansprüche 2 oder 3, dadurch gekennzeichnet, daß das Vernetzungsmonomer ein Diacryl- oder Dimethacryl-Monomer oder ein difunktionelles aromatisches Monomer ist.

5. Nagellack nach einem der Ansprüche 2 bis 4, dadurch gekennzeichnet, daß das Mikrogel durch Quellen des Polymers eines Mikrolatex in einem Lösungsmittel erhalten wird, das durch Radikalpolymerisation eines monofunktionellen Monomers in Anwesenheit eines Vernetzungsmonomers und eines grenzflächenaktiven Stoffs, vorzugsweise eines anionischen grenzflächenaktiven Stoffs, hergestellt wird.

6. Nagellack nach Anspruch 5, dadurch gekennzeichnet, daß der grenzflächenaktive Stoff ein makromolekularer grenzflächenaktiver Stoff ist.

7. Nagellack nach einem der Ansprüche 5 oder 6, dadurch gekennzeichnet, daß der grenzflächenaktive Stoff ein durch Carboxyl-Gruppen funktionalisierter Polyester ist.

8. Nagellack nach einem der Ansprüche 5 oder 6, dadurch gekennzeichnet, daß der grenzflächenaktive Stoff ein funktionalisiertes Polymer der Formel ist:

$$(P)\text{-}S\text{-}X\text{-}F \qquad (1),$$

worin

- (P) eine hydrophobe Polymer-Kette mit einer zahlenmittleren Molmasse zwischen 500 und 250 000 darstellt;
- S Schwefel ist;
- X bedeutet:
  - eine lineare oder verzweigte, gesättigte oder ungesättigte Kohlenwasserstoff-Kette mit 1 bis 6 Kohlenstoffatomen und substituiert durch zumindest eine Gruppe COOH oder $NH_2$, in freier oder versalzter Form,
  - eine Peptid-Kette, bestehend aus 2 bis 4 insbesondere natürlichen Aminosäuren,
- F eine Gruppe COOH oder $NH_2$, in freier oder versalzter Form, darstellt;

oder ein Polymer, das aus der Radikalpolymerisation zumindest eines Monomers in Anwesenheit eines Peptids erhalten wird, das zumindest eine Disulfid-Gruppe und/oder zumindest eine Thiol-Funktion trägt, wie ein Keratinhydrolysat.

9. Nagellack nach Anspruch 8, dadurch gekennzeichnet, daß das funktionalisierte Polymer aus der Radikalpolymerisation eines Monomers in Anwesenheit eines Thiols der Formel H-S-X-F oder eines Disulfids der Formel F-X-S-S-X-F erhalten wird, worin:

- X bedeutet:
  - eine lineare oder verzweigte, gesättigte oder ungesättigte Kohlenwasserstoff-Kette mit 1 bis 6 Kohlenstoffatomen und substituiert durch zumindest eine Gruppe COOH oder $NH_2$, in freier oder versalzter Form,
  - eine Peptid-Kette, bestehend aus 2 bis 4 insbesondere natürlichen Aminosäuren,
- F eine Gruppe COOH oder $NH_2$, in freier oder versalzter Form, darstellt;

wobei das Thiol oder Disulfid als Kettentransfermittel bei der Radikalpolymerisation wirkt, und wobei das (oder die) Monomer(e) zur Bildung der Polymer-Kette (P), wie in Anspruch 8 definiert, führt (oder führen).

10. Nagellack nach einem der Ansprüche 8 oder 9, dadurch gekennzeichnet, daß die Polymer-Kette (P) eine zahlenmittlere Molmasse von vorzugsweise weniger als 20 000 und vorzugsweise zwischen 500 und 10 000 aufweist.

11. Nagellack nach einem der Ansprüche 8 bis 10, dadurch gekennzeichnet, daß der Teil F-X, worin F und X die in Anspruch 8 angegebenen Bedeutungen haben, zumindest eine Carboxyl-Funktion und zumindest eine Amin-Funktion, in freier oder versalzter Form, umfaßt.

12. Nagellack nach einem der Ansprüche 8 bis 11, dadurch gekennzeichnet, daß das funktionalisierte Polymer die Formel aufweist:

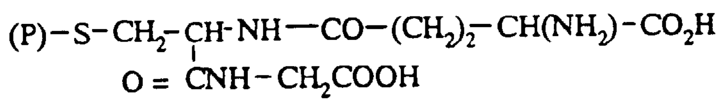

**13.** Nagellack nach Anspruch 12, dadurch gekennzeichnet, daß das funktionalisierte Polymer durch Radikalpolymerisation eines Monomers, das zur polymerisierten Kette, wie in Anspruch 8 definiert führt, in Anwesenheit eines aus Glutathion bestehenden Kettentransfermittels erhalten wird.

**14.** Nagellack nach einem der Ansprüche 8 bis 11, dadurch gekennzeichnet, daß das funktionalisierte Polymer durch Radikalpolymerisation zumindest eines Monomers in Anwesenheit von Cystein oder Homocystein, das als Kettentransfermittel wirkt, erhalten wird.

**15.** Nagellack nach einem der Ansprüche 8 bis 11, dadurch gekennzeichnet, daß das funktionalisierte Polymer durch Radikalpolymerisation zumindest eines Monomers in Anwesenheit eines Peptids, das zumindest eine Disulfid-Gruppe und/oder zumindest eine Thiol-Funktion trägt, wie ein Keratinhydrolysat, erhalten wird.

**16.** Nagellack nach einem der Ansprüche 5 oder 6, dadurch gekennzeichnet, daß der grenzflächenaktive Stoff ein am Kettenende funktionalisiertes Polymer der Formel ist:

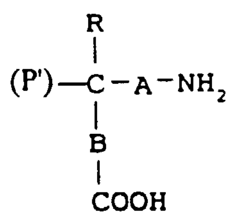

(II),

worin:

- die Polymer-Kette (P') eine hydrophobe Kette ist, die durch Radikalpolymerisation zumindest eines Monomers erhalten wird, und deren zahlenmittlere Molmasse zwischen 500 und 250 000, vorzugsweise unter 20 000, vorzugsweise zwischen 500 und 10 000 liegt;
- R bedeutet: ein Wasserstoffatom oder eine gerade oder verzweigte Kohlenwasserstoff-Kette mit 1 bis 8 Kohlenstoffatomen, gegebenenfalls substituiert durch zumindest eine Gruppe ausgewählt aus $CO_2H$, $NH_2$, OH oder einer Phenyl-Gruppe, die selbst gegebenenfalls substituiert ist;
- A und B, die gleich oder verschieden sind, jeweils eine Einfachbindung, eine gerade oder verzweigte, gesättigte oder ungesättigte Kohlenwasserstoff-Kette mit 1 bis 16 Kohlenstoffatomen, die eine Amid-Funktion enthalten kann, eine Peptid-Kette mit 2 bis 4 insbesondere natürlichen Aminosäuren bedeuten;
- die Gruppen COOH und/oder $NH_2$ frei oder versalzt sind.

**17.** Nagellack nach Anspruch 16, dadurch gekennzeichnet, daß das funktionalisierte Polymer eine der folgenden Formeln aufweist:

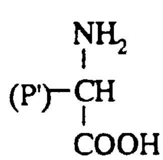

(3)

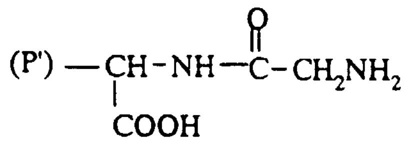

(4)

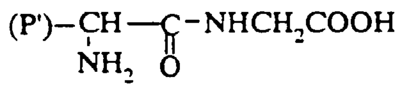

(5)

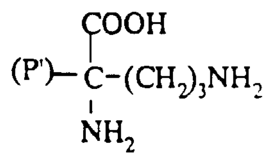

(6)

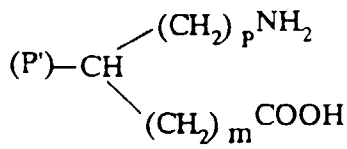

(7)

worin:

- (P') eine Polymer-Kette, wie in Anspruch 17 definiert, ist;
- m und p ganze Zahlen zwischen Null und 11 sind, deren Summe zwischen 2 und 11 beträgt;

oder ein funktionalisiertes Polymer ist, das durch Radikalpolymerisation zumindest eines Monomers in Anwesenheit einer Aminosäure oder eines Aminosäure-Derivats der Formel erhalten wird:

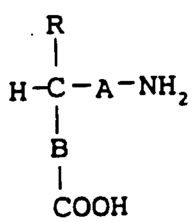

(2),

worin R, A und B die in Anspruch 17 angegebenen Bedeutungen haben.

**18.** Nagellack nach einem der Ansprüche 8 bis 17, dadurch gekennzeichnet, daß die Polymer-Kette aus der Radikalpolymerisation zumindest eines Acryl- oder Vinyl-Monomers erhalten wird.

**19.** Nagellack nach einem der Ansprüche 1 bis 18, dadurch gekennzeichnet, daß das Mikrogel aus Mikroteilchen besteht, die im aufgequollenen Zustand einen Durchmesser zwischen 10 und 300 nm, vorzugsweise zwischen 20 und 150 nm und vorzugsweise auch zwischen 30 und 100 nm aufweisen.

**20.** Nagellack nach einem der Ansprüche 1 bis 19, dadurch gekennzeichnet, daß er 1 bis 30 Masse-% Mikrogel, vorzugsweise 5 bis 20 %, enthält.

**21.** Nagellack nach Anspruch 20, dadurch gekennzeichnet, daß der Trocken-Extrakt 20 bis 50 Masse-% ausmacht.

**22.** Nagellack nach einem der Ansprüche 1 bis 21, dadurch gekennzeichnet, daß er 0 bis 30 Masse-% Nitrocellulose,

vorzugsweise 0 bis 20 %, enthält.

**23.** Nagellack nach einem der Ansprüche 1 bis 22, dadurch gekennzeichnet, daß er 0 bis 3 Masse-% organophilen Ton, vorzugsweise 0 bis 1,5 %, vorzugsweise auch 0 bis 0,5 %, enthält.

**24.** Verwendung eines Mikrogels, wie in einem der vorhergehenden Ansprüche definiert, als Mittel zur Modifikation der physikalischen Eigenschaften einer Nagellack-Zusammensetzung mit im wesentlichen organischen Lösungsmitteln und/oder der Eigenschaften des Films, der beim Aufbringen der Zusammensetzung erhalten wird.

**25.** Verwendung nach Anspruch 24, dadurch gekennzeichnet, daß das Mittel zur Verbesserung der Plastizität des Films bestimmt ist.

**26.** Verwendung nach Anspruch 24, dadurch gekennzeichnet, daß das Mittel zur Regulierung der Rheologie der Zusammensetzung bestimmt ist.

## Claims

**1.** Nail varnish which contains an essentially non-aqueous organic solvent, characterised in that it contains, besides the usual constituents of nail varnishes, at least one microgel, said microgel not being obtained from an acrylic polymer microdispersion prepared by a radical polymerisation of at least one acrylic monomer in the presence of a poly(methyl methacrylate) (PMMA)- and poly(tert-butyl acrylate) (PtBuA)-based block copolymer.

**2.** Nail varnish according to claim 1, characterised in that said microgel comprises a cross-linked polymer based on a monofunctional monomer known as main monomer and a difunctional monomer acting as cross-linking agent.

**3.** Nail varnish according to claim 2, characterised in that said main monomer is an acrylic or methacrylic, styrenic or vinylic ester monomer having a C1 to C18 alkyl group.

**4.** Nail varnish according to one of claims 2 or 3, characterised in that the cross-linking monomer is a diacrylic or dimethacrylic monomer, or a difunctional aromatic monomer.

**5.** Nail varnish according to one of claims 2 to 4, characterised in that said microgel is obtained by swelling, in a solvent of the polymer, a microlatex prepared by radical polymerisation of a monofunctional monomer in the presence of a cross-linking monomer and a surfactant, preferably an anionic surfactant.

**6.** Nail varnish according to claim 5, characterised in that the surfactant is a macromolecular surfactant.

**7.** Nail varnish according to one of claims 5 or 6, characterised in that said surfactant is a polyester functionalised with carboxylic groups.

**8.** The nail varnish according to one of claims 5 or 6, characterised in that said surfactant is a functionalised polymer of the formula:

$$(P)\text{-}S\text{-}X\text{-}F \quad (1)$$

in which:

(P) is a hydrophobic polymer chain of number average molar mass between 500 and 250,000,
S represents sulphur,
X represents :

- a saturated or unsaturated linear or branched hydrocarbon chain having 1 to 6 carbon atoms and substituted with at least one COOH or $NH_2$ group, in the free or salified form;
- a peptide chain constituted of 2 to 4 amino acids, particularly natural amino acids;

F represents a COOH or $NH_2$ group, in the free or salified form,

or a polymer resulting from the radical polymerisation of at least one monomer in the presence of a peptide bearing at least one disulphide group and/or at least one thiol function, such as a keratin hydrolysate.

**9.** The nail varnish according to claim 8, characterised in that said functionalised polymer results from the radical polymerisation of a monomer in the presence of a thiol of the formula H-S-X-F or a disulphide of formula F-X-S-S-X-F in which:

- X represents :
  - saturated or unsaturated linear or branched hydrocarbon chain having 1 to 6 carbon atoms and substituted with at least one COOH or $NH_2$ group, in the free or salified form,
  - a peptide chain constituted of 2 to 4 amino acids, particularly natural amino acids,
- F represents a COOH or $NH_2$ group, in the free or salified form, said thiol or disulphide acting as chain transfer agent during said radical polymerisation, said monomer(s) leading to the formation of the polymer chain (P) such as defined in claim 8.

**10.** The nail varnish according to one of claims 8 or 9, characterised in that the polymer chain (P) has a number average molar mass preferably lower than 20,000 and preferably between 500 and 10,000.

**11.** The nail varnish according to one of claims 8 to 10, characterised in that the F-X part, F and X having the meanings given in claim 8, comprises at least one carboxylic function and at least one amine function, in the free or salified form.

**12.** The nail varnish according to one of claims 8 to 11, characterised in that said functionalised polymer is of formula:

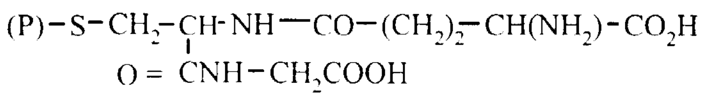

**13.** The nail varnish according to claim 12, characterised in that said functionalised polymer is obtained by radical polymerisation of a monomer leading to the polymerised chain such as defined in claim 8 in the presence of a chain transfer agent constituted by glutathione.

**14.** The nail varnish according to one of claims 8 to 11, characterised in that said functionalised polymer is obtained by radical polymerisation of at least one monomer in the presence of cysteine or homocysteine acting as chain transfer agent.

**15.** The nail varnish according to one of claims 8 to 11, characterised in that said functionalised polymer is obtained by radical polymerisation of at least one monomer in the presence of a peptide bearing at least one disulphide group and/or at least one thiol function, such as a keratin hydrolysate.

**16.** The nail varnish according to one of claims 5 or 6, characterised in that said surfactant is a chain end-functionalised polymer of the formula:

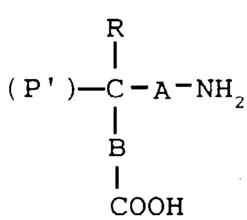

(II)

in which:

- the polymer chain (P') is a hydrophobic chain obtained by radical polymerisation of at least one monomer, and whose number average molar mass is between 500 and 250,000, preferably lower than 20,000, preferably between 500 and 10,000,
- R represents a hydrogen atom or a linear or branched hydrocarbon chain having 1 to 8 carbon atoms optionally substituted with at least one group selected from $CO_2H$, $NH_2$, OH or a phenyl group, itself being optionally substituted,
- A and B, identical or different. each represent a single bond. a saturated or unsaturated linear or branched hydrocarbon chain having from 1 to 16 carbon atoms, it being possible for it to contain an amide bond or a peptide chain having 2 to 4 amino acids, particularly natural amino acids,
- the $CO_2H$ and/or $NH_2$ groups being free or salified.

**17.** The nail varnish according to claim 16, characterised in that said functionalised polymer is of one of the following formulae:

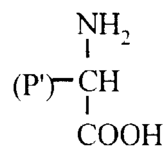
(3)

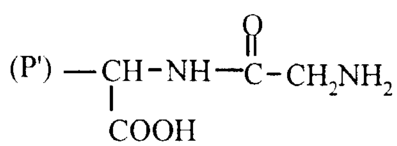
(4)

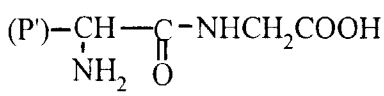
(5)

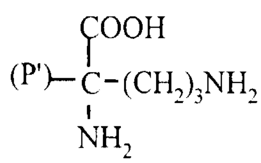
(6)

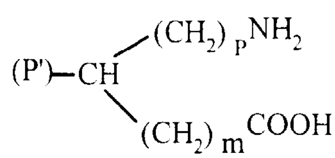
(7)

in which (P') is a polymer chain such as defined in claim 17,
m and p are integers between 0 and 11 and whose sum is between 2 and 11,

or a functionalised polymer obtained by radical polymerisation of at least one monomer in the presence of an amino acid or an amino acid derivative of the formula:

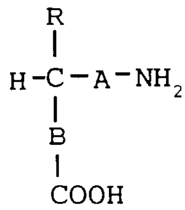

(2)

in which R, A and B have the meanings given in claim 17.

18. The nail varnish according to one of claims 8 to 17, characterised in that said polymer chain results from the radical polymerisation of at least one acrylic or vinylic monomer.

19. The nail varnish according to one of claims 1 to 18, characterised in that said microgel is constituted of microparticles which in the swollen state have a diameter between 10 and 300 nm, preferably between 20 and 150 nm, more preferably between 30 and 100 nm.

20. The nail varnish according to one of claims 1 to 19, characterised in that it contains from 1 to 30% by weight of microgel, preferably from 5 to 20%.

21. The nail varnish according to claim 20, characterised in that the dry extract represents from 20 to 50% by weight.

22. The nail varnish according to one of claims 1 to 21, characterised in that it contains from 0 to 30% by weight of nitrocellulose, preferably from 0 to 20%.

23. The nail varnish according to one of claims 1 to 22, characterised in that it contains from 0 to 3% by weight of organophilic clay, preferably from 0 to 1.5%, more preferably from 0 to 0.5%.

24. Use of a microgel such as defined in one of the preceding claims as an agent intended for modifying the physical properties of an essentially organic solvent nail varnish composition, and/or the properties of the film obtained during the application of said composition.

25. Use according to claim 24, characterised in that said agent is intended for improving the plasticity of said film.

26. Use according to claim 24, characterised in that said agent is intended for regulating the rheology of said composition.